# EUROPEAN PATENT APPLICATION

(11) **EP 3 088 389 A1**
(43) Date of publication of application: **02.11.2016**
(21) Application number: 15165465.4
(22) Date of filing: 28.04.2015
(51) Int. Cl.: C07D 211/46, A61K 31/445, A61P 3/00

(54) **4-EPI-ISOFAGOMINE DERIVATIVES**

(71) Applicant: Dorphan S.A., 1015 Lausanne (CH); Université d'Orléans, 45067 Orléans (FR)
(72) Inventor: Demotz, Stéphane, 1066 EPALINGES (CH); Martin, Olivier, 45160 ST HILAIRE ST MESMIN (FR); Boivineau, Estelle, 45240 LIGNY LE RIBAULT (FR); Front, Sophie, 45560 ST DENIS EN VAL (FR); Charollais-Thoenig, Julie, 1004 LAUSANNE (CH)
(74) Representative: KATZAROV S.A.

(57) **Abstract**

The present invention relates to 4-epi-isofagomine derivatives, pharmaceutically acceptable salts thereof, and the use of said 4-epi-isofagomine derivatives in the treatment and/or prevention of lysosomal storage diseases. The present invention is furthermore related to processes for their preparation.

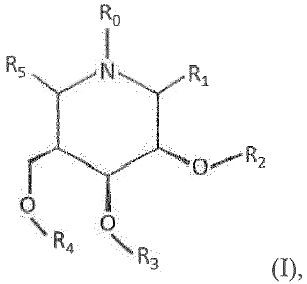

## Description

### TECHNICAL FIELD

The present invention relates to 4-epi-isofagomine derivatives, pharmaceutically acceptable salts thereof, and the use of said 4-epi-isofagomine derivatives in the treatment and prevention of lysosomal storage diseases. The present invention is furthermore related to processes for their preparation.

### BACKGROUND OF THE INVENTION

Lysosomal storage diseases constitute a group of approximately 50 rare inherited metabolic disorders that result from defects in lysosomal function, usually as a consequence of deficiency of a single enzyme required for the metabolism of lipids, glycoproteins or mucopolysaccharides.

Morquio disease type B, also called mucopolysaccharidosis IV type B (MPSIVB), and GM1-gangliosidosis are lysosomal storage diseases. They are caused by deficiencies of the beta-galactosidase (Brunetti-Pierri and Scaglia 2008, Caciotti et al. 2011, Sandhoff and Harzer 2013). In the absence of beta-galactosidase activity, keratan sulfate and GM1-ganglioside are not properly degraded and accumulate in the lysosomes, causing cell dysfunction and, ultimately, the diseases. In most cases, these two diseases appear early in life and in many cases the patients succumb before reaching adulthood. Morquio disease type B affects mainly the development of peripheral organs, such as skeleton and heart, with a limited involvement of the central nervous system, and GM1-gangliosidosis mainly affects brain development.

Different therapies have been exploited for the treatment of lysosomal storage diseases. The enzyme replacement therapy has been successfully exploited for the treatment of lysosomal storage diseases affecting mainly peripheral organs readily accessible to protein administered in the circulation. Notable examples of successful enzyme replacement drugs are Fabrazyme for the treatment of Fabry disease, Cerezyme, for the treatment of Gaucher Disease and Naglazyme for the treatment of mucopolysaccharidosis VI.

The hematopoietic stem cell transplantation has been successful used in severe cases of Hurler syndrome. This medical procedure, however, has not been found to alleviate symptoms in other mucopolysaccharidoses and, in addition, constitutes a high risk medical procedure (Noh and Lee 2014).

The substrate reduction therapy has been exploited to treat Gaucher disease with the development of the drugs miglustat and eliglustat. The therapy consists in limiting the synthesis of the cell constituent, which is no longer degraded due to an enzymatic deficiency. It follows that accumulation of the un-degraded cell constituent is slowed down, delaying onset and severity of the disease.

Iminosugars constitute a class of compounds, which has been extensively and successfully exploited for the development of potent and specific glycosidase inhibitors (Nash et al 2011). Attempts to develop iminosugars for the treatment of certain forms of Gaucher disease met insurmountable difficulties during clinical development. In 2009, Phase II clinical trials with the experimental drug afegostat, also known as isofagomine, failed to demonstrate sufficient therapeutic efficacy, and led to the termination of its development.

As of today, no curative treatment is available to fight lysosomal storage diseases related to beta-galactosidase enzyme dysfunction such as Morquio disease type B, and GM1-gangliosodosis. Patients are only offered palliative care. This situation means that there is a significant unmet medical need for efficient and selective compounds for the treatment of lysosomal storage diseases related to beta-galactosidase enzyme dysfunction. In particular, an efficient and selective compound having limited toxic effects and capable to pass the blood brain barrier is still awaited.

### SUMMARY OF THE INVENTION

The present invention provides a 4-epi-isofagomine derivative of general formula (I): 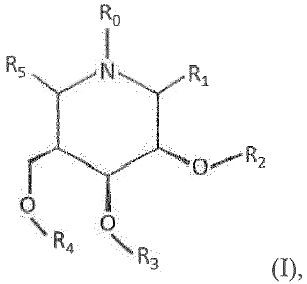 and pharmaceutically acceptable salts thereof wherein:
R₀ is selected from the group comprising hydrogen, C₁-C₃ alkyl, C₂-C₆ alkenyl, C₂-C₆ alkynyl, C₃-C₆ cycloalkyl, C₃-C₁₈ heterocycle, C₆ aryl, and C₇-C₈ arylalkyl;
R₁ is selected from the group comprising hydrogen, C₁-C₆ alkyl, C₂-C₆ alkenyl, C₂-C₆ alkynyl, C₃-C₆ cycloalkyl, C₃-C₁₈ heterocycle, C₆ aryl, and C₇-C₈ arylalkyl;
R₂, R₃ and R₄ are each independently selected from the group comprising hydrogen, C₁-C₄ alkyl, saturated or non-saturated C₁-C₄ acyl, C₂-C₄ alkenyl, and C₂-C₄ alkynyl;
R₅ is selected from the group comprising hydrogen, substituted or unsubstituted C₁-C₁₂ alkyl, C₂-C₁₂ alkenyl, C₂-C₁₂ alkynyl, C₃-C₁₂ cycloalkyl, C₃-C₁₈ heterocycle, C₆-C₁₈ aryl, C₇-C₁₈ arylalkyl, with the proviso that when R₅ is hydrogen, at least one of R₀, R₁, R₂, R₃ and R₄ is not hydrogen.

The invention also provides a 4-epi-isofagomine derivative of general formula (I), for use as a medicament. It further provides a 4-epi-isofagomine derivative of general formula (I), for use in the treatment and/or prevention of diseases caused by dysfunctions in beta-galactosidase. Also contemplated is a 4-epi-isofagomine derivative of general formula (I), for use in the treatment and/or prevention of Morquio disease type B, GM1-gangliosodosis, Krabbe disease, galactosemia, Tay-Sachs disease, Sandhoff-Jatzkewitz's disease, Fabry disease, metachromatic leukodystrophy, and/or Morquio disease type A.

Another object of the invention is to provide a 4-epi-isofagomine derivative of general formula (I), for use in the treatment and/or prevention of lysosomal storage diseases, wherein said lysosomal storage diseases are selected from the group of pathologies caused by dysfunctions in beta-galactosidase, alpha-galactosidase, galactocerebrosidase, alpha-glucosidase, alpha-mannosidase, beta-mannosidase, alpha-fucosidase galactose-1-phosphate uridylyltransferase, galactosylceramide beta-galactosidase and galactosyltransferases.

Furthermore, the invention provides pharmaceutical compositions comprising a 4-epi-isofagomine derivative of general formula (I) and a pharmaceutically acceptable carrier, diluent or excipient.

### BRIEF DESCRIPTION OF THE FIGURES

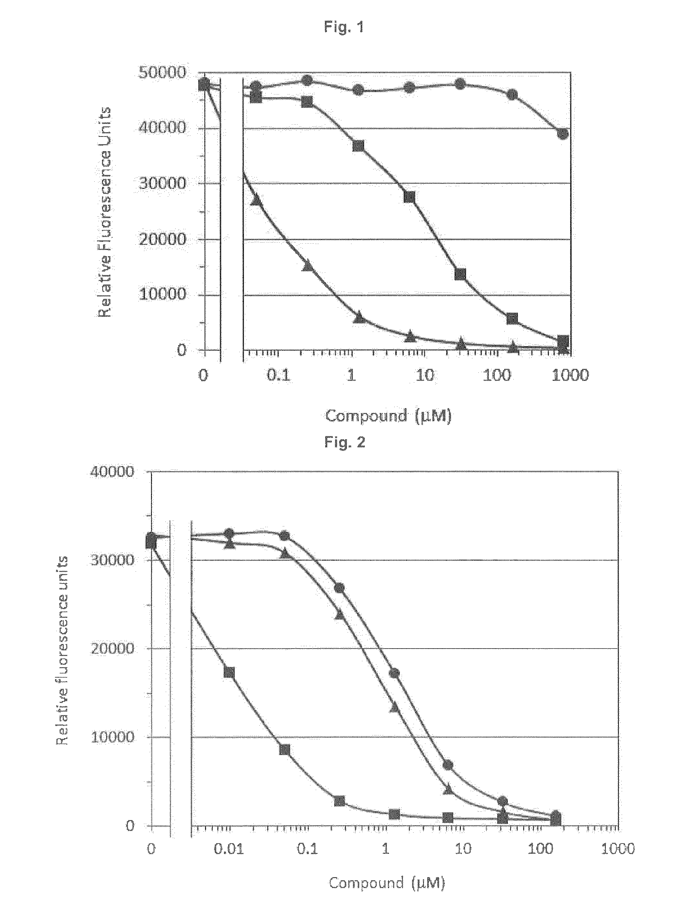

Figure 1: The beta-galactosidase activity in human peripheral blood mononuclear cell lysate was measured in the presence of increasing concentrations of N-nonyl-epi-isofagomine or epi-isofagomine. Fluorescence measurements at 445 nm, using an excitation wavelength of 365 nm, are correlated to the inhibition of beta-galactosidase obtained with N-nonyl-epi-isofagomine (squares), epi-isofagomine (triangles) and DMSO vehicle (circles).
Figure 2: The beta-galactosidase activity in human peripheral blood mononuclear cell lysate is inhibited in the presence of increasing concentrations of (2R,3R,4S,5R)-3-hydroxymethyl-2-pentylpiperidine-4,5-diol, (2R,3R,4S,5S)-3-hydroxymethyl-2-pentylpiperidine-4,5-diol, or epi-isofagomine. Fluorescence measurements at 445 nm, using an excitation wavelength of 365 nm, are correlated to the inhibition of beta-galactosidase by (2R,3R,4S,5R)-3-hydroxymethyl-2-pentylpiperidine-4,5-diol (squares), (2R,3R,4S,5S)-3-hydroxymethyl-2-pentylpiperidine-4,5-diol (circles) and epi-isofagomine (triangles).

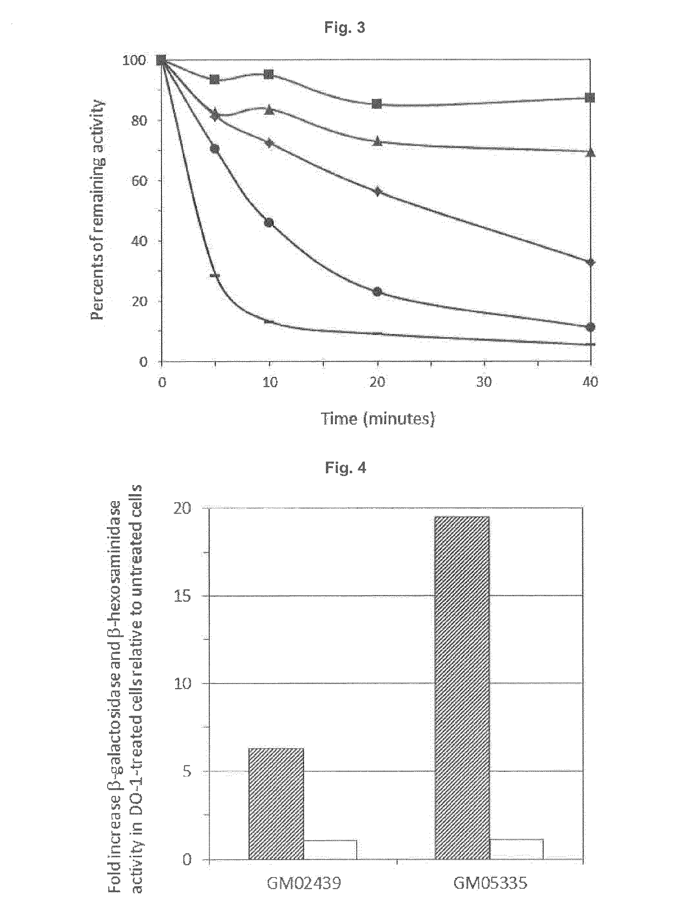

Figure 3: The human beta-galactosidase activity is stabilized in the presence of (2R,3R,4S,5R)-3-hydroxymethyl-2-pentylpiperidine-4,5-diol following heat denaturation. Samples of human peripheral blood mononuclear cell lysate were incubated at 48°C in the presence of graded concentrations of (2R,3R,4S,5R)-3-hydroxymethyl-2-pentylpiperidine-4,5-diol. Fluorescence was determined at 445 nm, using an excitation wavelength at 365 nm, and is correlated to the remaining beta-galactosidase activity. The tested concentrations of (2R,3R,4S,5R)-3-hydroxymethyl-2-pentylpiperidine-4,5-diol were 20 µM (square), 2 µM (triangle), 0.2 µM (diamond), 0.02 µM (circle) and 0 µM (line).
Figure 4: The beta-galactosidase and beta-hexosaminidase activity in fibroblasts of GM1-gangliosidosis patients is induced in the presence of (2R,3R,4S,5R)-3-hydroxymethyl-2-pentylpiperidine-4,5-diol. GM02439 and GM05335 fibroblasts (Coriell Institute) were cultured in the presence or the absence of 2 µM of (2R,3R,4S,5R)-3-hydroxymethyl-2-pentylpiperidine-4,5-diol. Beta-galactosidase and beta-hexosaminidase activities were determined in the cell lysates using fluorescence and colorimetric assays, respectively. Results of figure 4 represent the fold increase of beta-galactosidase or beta-hexosaminidase activities in (2R,3R,4S,5R)-3-hydroxymethyl-2-pentylpiperidine-4,5-diol-treated cells relative to untreated cells. Results are means of 4 values obtained from independent cultures. Percent of deviation of each value is <30% from the mean. Hatched and empty bars: beta-galactosidase and beta-hexosaminidase activities, respectively.

### DETAILED DESCRIPTION OF THE INVENTION

The present invention relates to a 4-epi-isofagomine derivative of general formula (I): 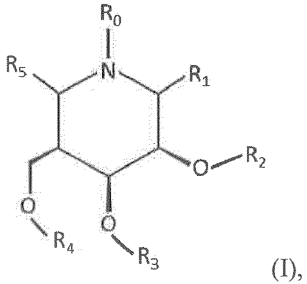 and pharmaceutically acceptable salts thereof wherein:
R₀ is selected from the group comprising hydrogen, C₁-C₃ alkyl, C₂-C₆ alkenyl, C₂-C₆ alkynyl, C₃-C₆ cycloalkyl, C₃-C₁₈ heterocycle, C₆ aryl, and C₇-C₈ arylalkyl;
R₁ is selected from the group comprising hydrogen, C₁-C₆ alkyl, C₂-C₆ alkenyl, C₂-C₆ alkynyl, C₃-C₆ cycloalkyl, C₃-C₁₈ heterocycle, C₆ aryl, and C₇-C₈ arylalkyl;
R₂, R₃ and R₄ are each independently selected from the group comprising hydrogen, C₁-C₄ alkyl, saturated or non-saturated C₁-C₄ acyl, C₂-C₄ alkenyl, and C₂-C₄ alkynyl;
R₅ is selected from the group comprising hydrogen, substituted or unsubstituted C₁-C₁₂ alkyl, C₂-C₁₂ alkenyl, C₂-C₁₂ alkynyl, C₃-C₁₂ cycloalkyl, C₃-C₁₈ heterocycle, C₆-C₁₈ aryl, C₇-C₁₈ arylalkyl, with the proviso that when R₅ is hydrogen, at least one of R₀, R₁, R₂, R₃ and R₄ is not hydrogen.

The following definitions are supplied in order to facilitate the understanding of the present invention.

As used herein, the term "comprise" is generally used in the sense of include, that is to say permitting the presence of one or more features or components.

As used herein, the singular form "a", "an" and "the" include plural references unless the context clearly dictates otherwise.

The following paragraphs provide definitions of the various chemical moieties that make up the compounds according to the invention and are intended to apply uniformly throughout the specification and claims unless an otherwise expressly set out definition provides a broader definition.

"C₁-C₁₂ alkyl" refers to monovalent straight-chained and branched alkyl groups having 1 to 12 carbon atoms, such as C₁-C₃ alkyl, C₁-C₄ alkyl or C₁-C₆ alkyl. Examples of straight chain alkyl groups include, but are not limited to, those with from 1 to 12 carbon atoms such as methyl, ethyl, n-propyl, n-butyl, n-pentyl, n-hexyl groups, n-heptyl, n-octyl, n-nonyl and n-decyl groups. Examples of branched alkyl groups include, but are not limited to, isopropyl, iso-butyl, sec-butyl, tert-butyl, isopentyl, and 2,2-dimethylpropyl groups. Alkyl groups may be substituted or unsubstituted. Representative substituted alkyl groups may be substituted one or more times with for example, amino, oxo, hydroxy, cyano, carboxy, nitro, thio, alkoxy, F, Cl, Br, I, cycloalkyl, aryl, heterocyclyl and heteroaryl groups.

"C₂-C₁₂ alkenyl" refers to straight and branched chain alkyl and cycloalkyl groups as defined above, except that at least one double bond exists between two carbon atoms. Thus, alkenyl groups have from 2 to 12 carbon atoms, such as C₂-C₆ alkenyl, C₂-C₄ alkenyl or C₂-C₁₀ alkenyl. Examples include, but are not limited to, vinyl, CH=CH(CH3), CH=C(CH3)2, C(CH3)=CH2, C(CH3)=CH(CH3), C(CH2CH3)=CH2, cyclohexenyl, cyclopentenyl, cyclohexadienyl, butadienyl, pentadienyl, and hexadienyl, among others. Alkenyl groups may be substituted or unsubstituted.

"C₂-C₁₀ alkynyl" refers to straight and branched chain alkyl groups, except that at least one triple bond exists between two carbon atoms. Thus, alkynyl groups have from 2 to 10 carbon atoms, such as C₂-C₆ alkynyl, C₂-C₄ alkynyl, or C₂-C₁₀ alkynyl. Examples include, but are not limited to, 1-ethynyl, 1-propynyl, 2-propynyl, 1-butynyl, 2-butynyl, 1-pentynyl or 2-pentynyl radical, among others. Alkynyl groups may be substituted or unsubstituted.

"C₃-C₁₂ cycloalkyl" refers to cyclic alkyl groups having from 3 to 12 carbon atoms such as, but not limited to C₃-C₆ cycloalkyl (cyclopropyl, cyclobutyl, cyclopentyl, cyclohexyl), cycloheptyl, cyclooctyl groups, and C₃-C₁₀ cycloalkyl. Cycloalkyl groups further include mono-, bicyclic and polycyclic ring systems, such as, for example bridged cycloalkyl groups as described below, such as, but not limited to, adamantyl, and fused rings, such as, but not limited to, decalinyl, and the like. Cycloalkyl groups may be substituted or unsubstituted. Cycloalkyl groups may be substituted one or more times with non-hydrogen groups as defined above (substituents). However, substituted cycloalkyl groups also include rings that are substituted with straight or branched chain alkyl groups as defined above. Representative substituted cycloalkyl groups may be mono-substituted or substituted more than once, such as, but not limited to, 2,2-, 2,3-, 2,4-2,5- or 2,6-disubstituted cyclohexyl groups, which may be substituted with any of the groups listed above, for example, methyl, amino, hydroxy, cyano, carboxy, nitro, thio, alkoxy, F, Cl, Br and I groups.

"C₃-C₁₈ heterocycle" refers to non-aromatic ring groups containing 3 or more ring members, of which one or more is a heteroatom such as, but not limited to, N, O and S. In some embodiments, the heterocyclyl group contains 1, 2, 3, or 4 heteroatoms. In some embodiments, heterocyclyl groups include 3 to 6, 10, 12, or 15 ring atoms. Heterocyclyl groups encompass unsaturated, partially saturated and saturated ring systems, such as, for example, imidazolinyl and imidazolidinyl groups. The phrase "heterocyclyl group" includes fused ring species including those comprising fused aromatic and non-aromatic groups, such as, for example, 2,3-dihydrobenzo[1,4]dioxinyl. The phrase also includes bridged polycyclic ring systems containing a heteroatom such as, but not limited to, quinuclidyl. Heterocyclyl groups may have other groups, such as alkyl, oxo or halo groups, bonded to one of the ring members. These are referred to as "substituted heterocyclyl groups." Heterocyclyl groups may be substituted or unsubstituted. Heterocyclyl groups include, but are not limited to, pyrrolidinyl, pyrrolinyl, imidazolinyl, imidazolidinyl, piperidinyl, piperazinyl, morpholinyl, pyrazolidinyl, tetrahydropyranyl, thiomorpholinyl, pyranyl, tetrahydrofuranyl, dihydrobenzofuranyl, dihydroindolyl, azabenzimidazolyl, benzothiadiazolyl, imidazopyridinyl, thianaphthalenyl, xanthinyl, guaninyl, tetrahydroquinolinyl, and 2,3-dihydrobenzo[1,4]dioxinyl. Representative substituted heterocyclyl groups may be mono-substituted or substituted more than once, such as, but not limited to, triazolyl, pyridinyl or morpholinyl groups, which are 1-, 2-, 3-, 4-, 5-, or 6-substituted, or disubstituted with various groups as defined above, including, but not limited to, alkyl, oxo, carbonyl, amino, alkoxy, cyano, and/or halo.

The term "aryl" refers to cyclic aromatic hydrocarbons that do not contain heteroatoms. Aryl groups include monocyclic, bicyclic and polycyclic ring systems. Thus, aryl groups include, but are not limited to C₆ aryl (such as phenyl, benzyl, tolyl, xylyl, benzyliden, benzoyl), C₆-C₁₈ aryl such as azulenyl, heptalenyl, biphenylenyl, indacenyl, fluorenyl, phenanthrenyl, triphenylenyl, pyrenyl, naphthacenyl, chrysenyl, biphenyl, anthracenyl, indenyl, indanyl, pentalenyl, naphthyl groups. The phenyl groups substituted or not, are particularly preferred. The term "aryl" includes groups containing fused rings, such as fused aromatic-aliphatic ring systems (e.g. indanyl, tetrahydronaphthyl, and the like). Aryl groups may be substituted or unsubstituted. Groups such as tolyl are referred to as substituted aryl groups. Representative substituted aryl groups may be mono-substituted or substituted more than once. For example, monosubstituted aryl groups include, but are not limited to, 2-, 3-, 4-, 5-, or 6-substituted phenyl or naphthyl groups, which may be substituted with groups such as those listed above.

"C₇-C₁₈ arylalkyl" refers to a radical of the alkyl type as defined above substituted by an aryl group as defined above, such as C₇-C₈ arylalkyl. The benzyl and phenethyl groups are particularly preferred.

"C₁-C₄ acyl" refers to the group -C(O)R where R includes C₁-C₆ alkyl, aryl, and heteroaryl.

According to the invention, the groups identified above may be substituted or unsubstituted. In general, the term "substituted" refers to a functional group, as defined below, in which one or more bonds to a hydrogen atom are replaced by a bond to a non-hydrogen atom. Substituted groups also include groups, in which one or more bonds to a hydrogen atom are replaced by one or more bonds, including double or triple bonds, to a heteroatom. In some embodiments, substituted groups have 1, 2, 3, 4, 5, or 6 substituents. Examples of substituent groups include, but are not limited to, halogens (*i.e.* F, Cl, Br and I), hydroxyls, alkoxy, alkenoxy, alkynoxy, aryloxy, aralkyloxy, heterocyclyloxy, and heterocyclylalkoxy groups; carbonyls (oxo); carboxyls; esters; ethers; urethanes; oximes; hydroxylamines; alkoxyamines; thiols; sulfides such as alkyl, alkenyl, alkynyl, aryl, aralkyl, heterocyclyl and heterocyclylalkyl sulfide groups; sulfoxides; sulfones; sulfonyls; sulfonamides; amines; N-oxides; hydrazines; hydrazides; hydrazones; azides; amides; ureas; amidines; guanidines; enamines; imides; isocyanates; isothiocyanates; cyanates; thiocyanates; imines; nitriles; alkyl, alkenyl, alkynyl, aryl, aralkyl, heteroaryl, heterocyclyl, heterocyclylalkyl and cycloalkyl groups.

Substituted ring groups such as substituted cycloalkyl, aryl, heterocyclyl and heteroaryl groups also include rings and fused ring systems in which a bond to a hydrogen atom is replaced with a bond to a carbon atom. Therefore, substituted cycloalkyl, aryl, heterocyclyl and heteroaryl groups may also be substituted with alkoxy, alkyl, alkenyl, and alkynyl groups as defined below.

Thus, the present invention relates to 4-epi-isofagomine derivatives of general formula (I) and pharmaceutically acceptable salts thereof that contain at least four to five asymmetric chiral carbon atoms in the subtituted piperidine cycle. Said 4-epi-isofagomine derivatives of general formula (I) exist as R and S-enantiomers. Enantiomers of 4-epi-isofagomine derivatives of general formula (I) are selected from the group comprising (1R or 1S, 2R, 3R, 4S, 5R or 5S) 4-epi-isofagomine derivatives, and (2R, 3R, 4S, 5R or 5S) 4-epi-isofagomine derivatives. Preferably, 4-epi-isofagomine derivatives of general formula (I) are selected from the group comprising (2R, 3R, 4S, 5R) 4-epi-isofagomine derivatives.

The invention also relates to salts of the 4-epi-isofagomine derivatives of general formula (I), pure or mixed, racemic mixtures, stereoisomers, for example C5-epimers and C1-epimers, hydrates, solvates, solid forms, chemical modified compounds, and/or mixtures thereof. Preferably, these salts are pharmaceutically acceptable. According to the present invention, pharmaceutically acceptable salts are produced from acidic inorganic or organic compounds, or alkaline inorganic or organic compounds. As used herein, the phrase "pharmaceutically acceptable salt" refers to a salt that retains the biological effectiveness of the free acids and bases of a specified compound and that is not biologically or otherwise undesirable.

4-epi-isofagomine derivatives of general formula (I) and pharmaceutically acceptable salts thereof are stable in several solid forms. The present invention includes all the solid forms of the compounds according to the invention which includes amorphous, polymorphous, mono- and polycrystalline forms.

4-epi-isofagomine derivatives of general formula (I) and pharmaceutically acceptable salts thereof can also exist in non-solvated or solvated form, for example with pharmaceutically acceptable solvents such as water (hydrates) or ethanol. In particular, the invention relates to 4-epi-isofagomine derivatives of general formula (I), 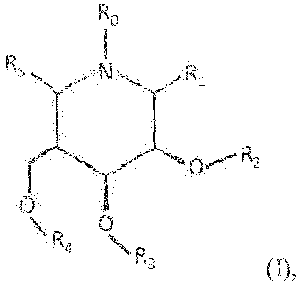 and pharmaceutically acceptable salts thereof, wherein:
R₀ is hydrogen;
R₁ is hydrogen;
R₂, R₃ and R₄ are each independently selected from the group comprising C₁-C₄ alkyl, saturated or non-saturated C₁-C₄ acyl, C₂-C₄ alkenyl, and C₂-C₄ alkynyl; and
R₅ is selected from the group comprising hydrogen, substituted or unsubstituted C₁-C₁₂ alkyl, C₂-C₁₂ alkenyl, C₂-C₁₂ alkynyl, C₃-C₁₂ cycloalkyl, C₃-C₁₈ heterocycle, C₆-C₁₈ aryl, C₇-C₁₈ arylalkyl.

Preferably, R₅ is selected from the group comprising pentyl, hexyl, heptyl, octyl, nonyl, ethylphenyl, 2-(4-trifluoromethylphenyl)ethyl, 2-ethylhexyl, cyclopropylmethyl, cyclohexylmethyl, cycloheptylmethyl, 5-hexenyl, 5-hexinyl, 5-acetamido-1-hydroxypent-2-yl, and 6-acetamido-hexyl.

In a preferred embodiment, the invention relates to 4-epi-isofagomine derivatives of general formula (I), 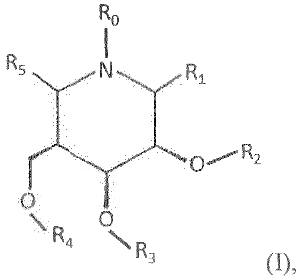 and pharmaceutically acceptable salts thereof, wherein R₀, R₁, R₂, R₃ and R₄ are hydrogen and R₅ is selected from the group comprising substituted or unsubstituted C₁-C₁₂ alkyl, C₂-C₁₂ alkenyl, C₂-C₁₂ alkynyl, C₃-C₁₂ cycloalkyl, C₃-C₁₈ heterocycle, C₆-C₁₈ aryl, C₇-C₁₈ arylalkyl.

Preferably, R₅ is selected from the group comprising pentyl, hexyl, heptyl, octyl, nonyl, ethylphenyl, 2-(4-trifluoromethylphenyl)ethyl, 2-ethylhexyl, cyclopropylmethyl, cyclohexylmethyl, cycloheptylmethyl, 5-hexenyl, 5-hexinyl, 5-acetamido-1-hydroxypent-2-yl, and 6-acetamido-hexyl.

More preferably, the 4-epi-isofagomine derivatives of general formula (I), and pharmaceutically acceptable salts thereof, are selected from the group comprising:
(2R,3R,4S,5R)-3-Hydroxymethyl-2-pentylpiperidine-4,5-diol;
(2R,3R,4S,5R)-3-Hydroxymethyl-2-hexylpiperidine-4,5-diol;
(2R,3R,4S,5R)-3-Hydroxymethyl-2-heptylpiperidine-4,5-diol;
(2R,3R,4S,5R)-3-Hydroxymethyl-2-octylpiperidine-4,5-diol;
(2R,3R,4S,5R)-3-Hydroxymethyl-2-nonylpiperidine-4,5-diol;
(2R,3R,4S,5R)-3-Hydroxymethyl-2-ethylphenylpiperidine-4,5-diol;
(2R,3R,4S,5R)-3-Hydroxymethyl-2-[2-(4-trifluoromethylphenyl)ethyl]piperidine-4,5-diol;
(2R,3R,4S,5R)-3-Hydroxymethyl-2-(2-ethylhexyl)piperidine-4,5-diol;
(2R,3R,4S,5R)-3-Hydroxymethyl-2-cyclopropylmethylpiperidine-4,5-diol;
(2R,3R,4S,5R)-3-Hydroxymethyl-2-cyclohexylmethylpiperidine-4,5-diol;
(2R,3R,4S,5R)-3-Hydroxymethyl-2-cycloheptylmethylpiperidine-4,5-diol;
(2R,3R,4S,5R)-3-Hydroxymethyl-2-(5-hexenyl)piperidine-4,5-diol;
(2R,3R,4S,5R)-3-Hydroxymethyl-2-(5-hexinyl)piperidine-4,5-diol;
(2R,3R,4S,5R)-3-Hydroxymethyl-2-(5-acetamido-1-hydroxypent-2-yl)piperidine-4,5-diol; and
(2R,3R,4S,5R)-3-Hydroxymethyl-2-(6-acetamido-hexyl)piperidine-4,5-diol.

According to a preferred embodiment, the 4-epi-isofagomine derivatives of general formula (I), and pharmaceutically acceptable salts thereof, is (2R,3R,4S,5R)-3-Hydroxymethyl-2-pentylpiperidine-4,5-diol of formula:

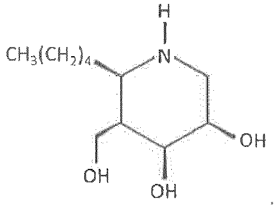

The present invention provides 4-epi-isofagomine derivatives of general formula (I) and pharmaceutically acceptable salts thereof, wherein said 4-epi-isofagomine derivatives are inhibitors of beta-galactosidase and have an IC50 of less than about 50 µM, preferably of less than about 10 µM, more preferably of less than about 5 µM.

As shown in the examples, (2R,3R,4S,5R)-3-hydroxymethyl-2-pentylpiperidine-4,5-diol has an IC50 of 0.01µM, and (2R,3R,4S,5S)-3-hydroxymethyl-2-pentylpiperidine-4,5-diol has an IC50 of 1.5 µM (Example 3).

Generally, the beta-galactosidase is a hydrolase enzyme that cleaves beta-linked terminal galactosyl residues from different substrates, including gangliosides, glycoproteins and glycosaminoglycans. Preferably, the beta-galactosidase is a lysosomal beta-galactosidase also called acid beta-galactosidase, as it refers to a protein located in the acidic environment of the lysosomes, while other beta-galactosidase activities are found in neutral environments (e.g. cytoplasm). The lysosomal beta-galactosidase is the product of the *GLB1* gene.

As used herein, the term "beta-galactosidase" refers to the enzyme from mammalian origin, preferably human beta-galactosidase.

As used herein, the term "inhibitor" refers to compounds that block or partially block, directly or indirectly the activity of an enzyme.

As used herein, the term "IC50" represents the concentration of a drug that is required for 50% inhibition of the enzyme activity.

As used herein, the term "about" applies to numeric values and refers to a range of numbers that one of skill in the art would consider equivalent to the recited values. For example, "about 10 µM" refers to 10 µM ± 10%.

Advantageously, the 4-epi-isofagomine derivatives of general formula (I) are highly specific inhibitors of beta-galactosidase activity.

As shown in the examples, (2R,3R,4S,5R)-3-hydroxymethyl-2-pentylpiperidine-4,5-diol is a highly specific beta-galactosidase inhibitor (IC50 = 0.02 µM), whereas it considerably less potently inhibits the human beta-glucosidase and the human alpha-galactosidase (IC50 = 60 µM and 150 µM, respectively), and does not inhibit any of the other four human glycosidases tested (IC50 > 160 µM) (Example 4).

Generally, pharmacological chaperones are small molecular weight compounds which tightly bind in the endoplasmic reticulum to newly synthesized proteins and favor them to adopt a native conformation. Once this status is attained, the protein is no longer retained in the endoplasmic reticulum for early degradation and traffics to its normal intracellular or extracellular destination. In the case of mutated proteins, which do not normally adopt a sufficiently native conformation to be cleared by the quality control system, the binding of chaperone compounds is believed to promote further maturation, avoiding the polypeptide to undergo premature degradation. According to this model, the mutated protein reaches its normal cellular compartment (e.g. lysosomes, plasma membranes and secretion in the extracellular space), where it can perform its functions. This mechanism of action relies on the fact that in many situations, mutations do not affect the catalytic activity of the protein, meaning that if the pharmacological chaperone compounds allow the transport of the mutated proteins to its normal cellular destination, it would partly restore the physiological enzymatic activity (Fan et al 1999, Boyd et al 2013).

In the context of the present invention, in addition to be specific inhibitors of beta-galactosidase activity, the 4-epi-isofagomine derivatives of general formula (I) are chaperone molecules of deficient mutated versions of the beta-galactosidase protein.

As used herein, the term "chaperone molecule" refers to small molecular weight compounds which bind to proteins to prevent loss of their native conformation, or favor them to adopt or restore a conformation ressembling the physiologically native one.

As used herein, the term "deficient" refers to totally or partially inactivated enzymes resulting from mutations, or denaturation through exposure to chemicals (*e*.*g*. urea and guanidine hydrochloride) or physical factors (e.g. heat and freeeze and thaw cycles).

Thus, the present invention provides 4-epi-isofagomine derivatives of general formula (I) and pharmaceutically acceptable salt thereof, wherein said 4-epi-isofagomine derivatives prevent denaturation of beta-galactosidase.

As used herein, the term "denaturation" refers to a process in which the folding conformation of a protein is altered due to exposure to certain chemical or physical factors (e.g. heat, acid, solvents, etc.), causing the protein to become biologically inactive.

As shown in the examples, following heat denaturation of peripheral blood mononuclear cell lysate at 48°C for 10 minutes, 84% of the beta-galactosidase activity was spared by the presence of 2 µM of (2R,3R,4S,5R)-3-hydroxymethyl-2-pentylpiperidine-4,5-diol, while this percentage was reduced to 13% in the absence of compound (Example 5, Figure 3).

The present invention also relates to 4-epi-isofagomine derivatives of general formula (I) and pharmaceutically acceptable salt thereof, for use as a medicament.

It further relates to 4-epi-isofagomine derivatives of general formula (I) and pharmaceutically acceptable salt thereof, for use in the treatment and/or prevention of Morquio disease type B, GM1-gangliosidosis, Krabbe disease, Tay-Sachs disease, Sandhoff-Jatzkewitz's disease, Fabry disease, metachromatic leukodystrophy, Morquio disease type A and/or galactosemia.

Morquio disease type B and GM1-gangliosidosis, are lysosomal storage diseases associated with beta-galactosidase enzyme dysfunctions.

Krabbe disease, Tay-Sachs disease, Sandhoff-Jatzkewitz's disease, Fabry disease, metachromatic leukodystrophy, Morquio disease type A and galactosemia are metabolic diseases caused by deficiencies of enzymatic activities necessary for the disposal of cell constituents containing at least at one point in their catabolic pathways terminal galactosyl residues.

For example, human beta-galactosidase gene mutations have been found in patients with Morquio disease type B and GM1-gangliosidosis.

Another object of the present invention relates to 4-epi-isofagomine derivatives of general formula (I) and pharmaceutically acceptable salt thereof, for use in the treatment and/or prevention of lysosomal storage diseases, wherein said lysosomal storage diseases are selected from the group of pathologies caused by deficiencies in beta-galactosidase, alpha-galactosidase, galactocerebrosidase, alpha-glucosidase, alpha-mannosidase, beta-mannosidase, alpha-fucosidase galactose-1-phosphate uridylyltransferase, galactosylceramide beta-galactosidase and galactosyltransferases. Preferably, the lysosomal storage diseases are related to beta-galactosidase enzyme dysfunctions.

Surprisingly, the 4-epi-isofagomine derivatives of general formula (I), (2R,3R,4S,5R)-3-hydroxymethyl-2-pentylpiperidine-4,5-diol induces increase of mutated beta-galactosidase activity in cell lines of patients suffering from lysosomal storage diseases associated with beta-galactosidase enzyme dysfunctions, such as GM1-gangliosidosis. The beta-galactosidase activity following treatment with 4-epi-isofagomine derivatives of general formula (I) is at least 2-fold, preferably 5-fold higher than in the absence of treatment.

As shown in the example, the 4-epi-isofagomine derivative, (2R,3R,4S,5R)-3-hydroxymethyl-2-pentylpiperidine-4,5-diol, induces increase of the mutated beta-galactosidase activity by 6.5- and 19-fold in GM1-gangliosidosis cell lines GM02439 and GM05335, respectively (Example 6 and Fig. 4). In contrast, there is no increase of the beta-hexosaminidase activity at all.

The present invention further relates to a pharmaceutical composition comprising 4-epi-isofagomine derivatives of general formula (I) and pharmaceutically acceptable salts thereof, and a pharmaceutically acceptable carrier, diluent or excipient.

The pharmaceutical composition of the invention is formulated in accordance with standard pharmaceutical practice (see, e.g., Remington: The Science and Practice of Pharmacy (20th ed.), ed. A. R. Gennaro, Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art. The excipient of the composition can be any pharmaceutically acceptable excipient, including specific carriers able to target specific cells or tissues. As stated earlier, possible pharmaceutical compositions include those suitable for oral, rectal, topical, transdermal, buccal, sublingual, or parenteral (including subcutaneous, intramuscular, intravenous and intradermal) administration. For these formulations, conventional excipients can be used according to techniques well known by those skilled in the art. The compositions for parenteral administration are generally physiologically compatible sterile solutions or suspensions which can optionally be prepared immediately before use from solid or lyophilized form. For oral administration, the composition can be formulated into conventional oral dosage forms such as tablets, capsules, powders, granules and liquid preparations such as syrups, elixirs, and concentrated drops. Non-toxic solid carriers or diluents may be used which include, for example, pharmaceutical grades of mannitol, lactose, starch, magnesium stearate, sodium saccharine, talcum, cellulose, glucose, sucrose, magnesium, carbonate, and the like. For compressed tablets, binders, which are agents which impart cohesive qualities to powdered materials, are also necessary. For example, starch, gelatine, sugars such as lactose or dextrose, and natural or synthetic gums can be used as binders. Disintegrants are also necessary in the tablets to facilitate break-up of the tablet. Disintegrants include starches, clays, celluloses, algins, gums and cross-linked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants. For transdermal administration, the composition can be formulated into ointment, cream or gel form and appropriate penetrants or detergents could be used to facilitate permeation, such as dimethyl sulfoxide, dimethyl acetamide and dimethylformamide. For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate. In a preferred embodiment, the pharmaceutical composition of the invention is suitable for parenteral administration.

Pharmaceutical composition according to the invention may be formulated to release the active drug substantially immediately upon administration or at any predetermined time or a time period after administration.

In a particular embodiment, the pharmaceutical composition according to the invention comprises 0.001 mg to 5 g of the compound of the invention. Preferably, pharmaceutical composition according to the invention comprises 0.01 mg to 1 g of the compound of the invention.

Pharmaceutical compositions according to the invention can comprise one or more compound of the invention in association with pharmaceutically acceptable excipients and/or carriers. These excipients and/or carriers are chosen according to the form of administration as described above.

The invention also relates to a pharmaceutical composition comprising 4-epi-isofagomine derivatives of general formula (I) and pharmaceutically acceptable salts thereof, and further comprising at least one or more additional agents selected from the group comprising non-competitive chaperone compounds and GM1-ganglioside synthesis inhibitors.

Non-competitive chaperone compounds are selected from the group comprising substances selected for their capacity to promote rescue of mutated forms of beta-galactosidase without inhibiting their enzymatic activity, a feature explained by the binding of these substances to the beta-galactosidase outside its catalytic site.

GM1-ganglioside synthesis inhibitors are selected from the group comprising for instance d-*threo*-1-phenyl-2-palmitoylamino-3-pyrrolidino-1-propanol (Tifft & Proia 2000) and N-butyldeoxygalactonojirimycin (Baek et al 2008).

The invention further relates to a process for preparing and isolating a 4-epi-isofagomine derivative of general formula (I) comprising the steps described in the scheme below starting from D-lyxose:

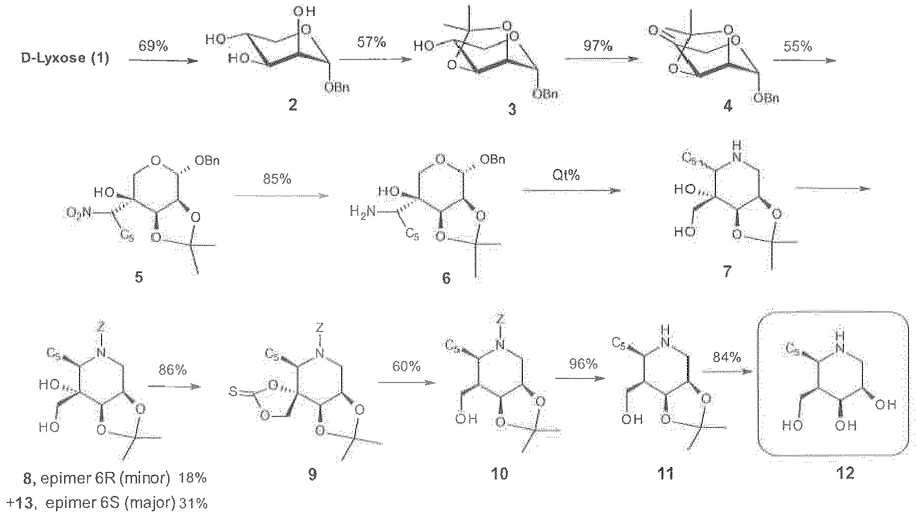

In particular, the invention relates to a process for preparing and isolating a 4-epi-isofagomine derivative comprising the steps of:
1) modification of D-lyxose **(1)** with benzyl alcohol to afford benzyl α-D-lyxopyranoside **(2),**
2) modification of **2** with dimethoxypropane to afford benzyl 2,3-*O*-isopropylidene-α-D-lyxopyranoside **(3),**
3) reaction of **3** with Dess-Martin periodinane, resulting in benzyl 2,3-*O*-isopropylidene-β-L-*erythro*-pent-4-uloside **(4),**
4) reaction between **4** and 1-nitrohexane to produce benzyl 2,3-*O*-isopropylidene-4-*C-*[(1'*R*,1'*S*)(1-nitrohex-1-yl)]-β-L-ribopyranosides **(5),**
5) reduction of **5** with Raney nickel to afford benzyl 2,3-*O*-isopropylidene--[(1'*R*,1'*S*)(1-aminohex-1-yl)]-β-L-ribopyranosides **(6),**
6) rearrangement of **6** catalyzed by palladium hydroxide in the presence of hydrogen, affording *(3R, 4R,* 5*S, 6R*/*6S*)-5-hydroxymethyl-3,4-*O*-isopropylidene-6-*C*-pentyl-piperidine-3,4,5-triol **(7)**,
7) reaction of **7** with benzyl chloroformate to obtain (3*R*,4*R*,5*S*,6*R*)-1-benzyloxycarbonyl-5-hydroxymethyl-3,4-*O*-isopropylidene-6-*C*-pentyl-piperidine-3,4,5-triol **(8),**
8) modification of **8** with thiocarbonyldiimidazole to afford 3*R*,4*R*,5*S*,6*R*)-1-benzyloxycarbonyl-5-hydroxymethyl-3,4-*O*-isopropylidene-6-*C*-pentyl-piperidine-3,4,5-triol, 5,5'-thiocarbonyl derivative **(9),**
9) modification of **9** by reaction with tributyltin hydride to obtain (3*R*,4*R*,5*R*,6*R*)-1-benzyloxycarbonyl-5-hydroxymethyl-3,4-*O*-isopropylidene-6-*C*-pentyl-piperidine-3,4-diol **(10),**
10) deprotection of **10** with palladium in the presence of hydrogen to produce (3*R*,4*R*,5*R*,6*R*)-5-hydroxymethyl-3,4-*O*-isopropylidene-6-*C*-pentyl-piperidine-3,4-diol **(11),** and
11) treatment of **11** with an acidic ion-exchange resin in dioxane and H₂O to obtain (3*R*,4*R*,5*R*,6*R*)-5-hydroxymethyl-6-*C*-pentyl-piperidine-3,4-diol **12**.

In particular, important steps in the process for preparing and isolating a 4-epi-isofagomine derivative from D-lyxose are the modification of 2,3-*O*-isopropylidene-β-L-*erythro*-pent-4-uloside with 1-nitroalkane to produce benzyl 2,3-*O*-isopropylidene-4-*C*-[(1'*R*,1'*S*)(1-nitroalk-1-yl)]-β-L-ribopyranosides and, production ultimately of (3*R*,4*R*,5*R*,6*R*)-5-hydroxymethyl-6-*C-*alkyl-piperidine-3,4-diol.

### EXAMPLES

### Example 1: Inhibition of wild type beta-galactosidase activity in human peripheral blood mononuclear cell lysate by N-nonyl-epi-isofagomine and epi-isofagomine.

### Experimental Protocol

The Applicant has synthesized a compound of formula N-nonyl-epi-isofagomine wherein the nitrogen atom of epi-isofagomine was derivatized with a nonyl chain. 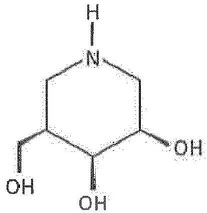 4-epi-isofagomine (Isofagomine (3R,4R,5R)-5-(hydroxymethyl)-3,4-piperidinediol)

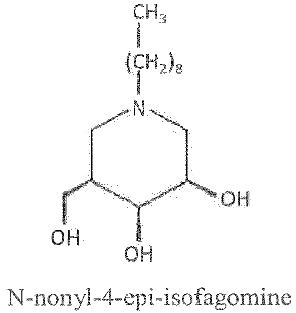

Thus, these compounds do not belong to the group of derivatives comprising 4-epi-isofagomine derivatives of general formula (I).

Samples of human peripheral blood mononuclear cell lysate (2 x 10⁶ cells per assay) were incubated with graded concentrations of compound N-nonyl-4-epi-isofagomine or 4-epi-isofagomine in 50 mM sodium phosphate, pH 7.3, supplemented with 1% Triton X-100 and protease inhibitors, and 0.8 mM of 4-methylumbelliferyl β-D-galactopyranoside. After an incubation of 2 hours at 37°C, the enzymatic reactions were stopped by the addition of 0.15 M glycine, pH 10.2, and fluorescence was measured at 445 nm using an excitation wavelength of 365 nm. The stock solutions of compound were at 20 mM in DMSO.

The results obtained with N-nonyl-4-epi-isofagomine (squares), 4-epi-isofagomine (triangles) and DMSO vehicle (circles) are presented on figure 1.

### Results

The N-nonyl-4-epi-isofagomine derivative that is not a 4-epi-isofagomine derivative of general formula (I) is 140-fold less inhibitory on human beta-galactosidase than 4-epi-isofagomine (Fig. 1). In this experiment, the IC50 of 4-epi-isofagomine is 0.07 µM and the IC50 N-nonyl-4-epi-isofagomine is 10 µM.

### Example 2: Synthesis of 4-epi-isofagomine derivatives of general formula (I).

Extensive medicinal chemistry efforts around the epi-isofagomine scaffold resulted in the identification of (2R,3R,4S,5R)-3-hydroxymethyl-2-pentylpiperidine-4,5-diol as a promising compound.

The process for preparing and isolating a 4-epi-isofagomine derivative according to the invention starts from D-lyxose, and comprises the 11 steps described in details below.

### Benzyl α-D-lyxopyranoside 2

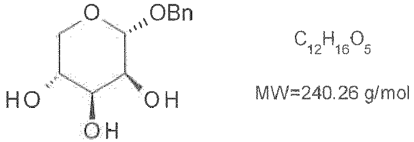

To a solution of D-lyxose (20.0 g, 0.13 mol) in benzyl alcohol (37 mL) was added *p-*toluenesulfonic acid (201 mg, 1.10 mmol, 0.008 eq). The reaction was left stirring for 78 h at 60°C. Then the mixture was cooled down to RT, concentrated under vacuum and co-evaporated with toluene (3 x 30 mL) to give a soft white solid. This residue was slurred into a mixture of hexane/CH₂Cl₂ (2:1) (180 mL). The solid was filtered, then stirred with cold Et₂O (25 mL), filtered again and dried under vacuum. The filtrate was concentrated and the procedure was repeated three times to give the desired compound **2** (21.6 g, 69%) as a white solid. The obtained NMR-spectrum is consistent with literature data (Keck, G. E.; Kachensky, D. F.; Enholm, E. J. J Org Chem 1985, 50, 4317).

### Benzyl 2,3-O-isopropylidene-α-D-lyxopyranoside 3

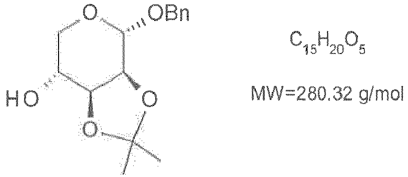

The reaction was conducted under Ar. To a solution of compound **2** (9.62 g, 0.04 mol) in acetone (128 mL) were added dimethoxypropane (16.7 mL, 0.14 mol, 3.5 eq) and *p*-toluenosulfonic acid (150 mg, 0.8 mmol, 0.02 eq). The reaction was left stirring overnight at RT. The mixture was concentrated under vacuum and the residue was diluted with EtOAc (120 mL). The solution was washed with saturated aqueous NaHCO₃ (40 mL), water (2 x 40 mL) and dried over MgSO₄. Concentration under vacuum gave crude **3** as a colorless oil. Flash column chromatography of the crude product on silica gel (PE/EtOAc 85:15/5:5) afforded compound **3** (6.46 g, 57%) as a white solid. The obtained NMR-spectrum is consistent with literature data (same ref. as **2).**

### Benzyl 2,3-O-isopropylidene-β-L-erythro-pent-4-uloside 4

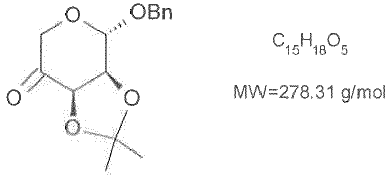

The reaction was conducted under Ar. To a solution of alcohol **3** (1.0 g, 3.57 mmol) in anhydrous CH₂Cl₂ (36 mL) was added Dess-Martin periodinane (1.82 g, 4.28 mmol, 1.2 eq). The reaction was left stirring overnight at RT. The solvent was then evaporated under vacuum. The residue was suspended in the minimal volume of cold Et₂O and the solids were removed by filtration through a membrane. The filtrate was concentrated under vacuum to give the crude product as a colorless oil with traces of a white solid. Rapid filtration of the product through silica gel (PE/EtOAc 8:2) gave the desired ketone **4** (0.98 g, 97%) as a colorless oil. The NMR spectrum of **4** is consistent with literature data (Anastasi, C.; Buchet, F. F.; Crowe, M. A.; Helliwell, M.; Raftery, J.; Sutherland, J. D. Chem. Eur. J. 2008,14,2375).

### Benzyl 2.3-O-isopropylidene-4-C-[(1'R.1'S)(1-nitrohex-1-yl)]-β-L-ribopyranosides 5a and 5b

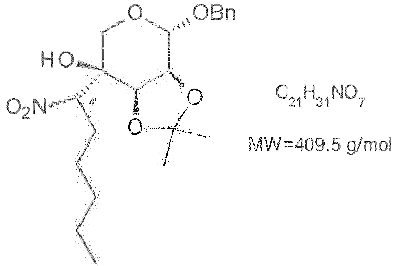

To a solution of ketone **4** (1.98 g, 7.11 mmol) in 1-nitrohexane (5.5 mL) was added triethylamine (2.8 mL, 20.2 mmol, 2.8 eq). The mixture was left stirring for 4 d at RT. The reaction was quenched with saturated aqueous NH₄Cl (40 mL). The aqueous phase was separated and extracted with EtOAc (80 mL). The organic phase was then washed with brine (40 mL) and dried over MgSO₄. Concentration of the organic phase under vacuum gave the crude product. Flash column chromatography on silica gel (PE/EtOAc, 92:8) gave an inseparable mixture of the two diastereoisomers at 4'-position in a 7:3 ratio, **5a** and **5b** (1.5 g, 55%), as a colorless oil.

### Numbering of compound for NMR data

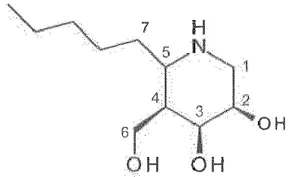

¹H NMR (400 MHz, isomers labelled as MAJ and min, CDCl₃), 8 7.47-4.28 (m, 5H, Hₐᵣₒₘ), 5.05 (br s, 1H, H1), 4.75 (d, 1H, CH₂Ph, *J =* 11.6 Hz), 4.60-4.51 (m, 1H, H6), 4.54 (d, 1H, CH₂Ph, *J* = 11.6 Hz), 4.26 (d, 0.7H, H3_{MAJ}, *J* = 6.4 Hz), 4.17-4.09 (m, 1.3H, H2_{MAJ}, H3ₘᵢₙ, H2ₘᵢₙ), 3.88 (d, 0.3H, H5_{bmin}, *J* = 12.4 Hz), 3.81 (d, 0.7H, H5_{bMAJ}, *J=* 12.0 Hz), 3.73 (d, 0.7H, H5_{aMAJ}, *J =* 12.0 Hz), 3.58 (d, 0.3H, H5ₐₘᵢₙ, *J* = 12.4 Hz), 3.04 (s, 0.7H, OH_{MAJ}), 2.96 (s, 0.3H, OHₘᵢₙ), 2.16-1.95 (m, 1.3H, H7_{bMAJ}, 2H7ₘᵢₙ), 1.72-1.65 (m, 0.7H, H7_{aMAJ}), 1.55 (s, 0.9H, CH₃iPrₘᵢₙ), 1.54 (s, 2.1H, CH₃iPr_{MAJ}), 1.37 (s, 0.9H, CH₃iPrₘᵢₙ), 1.36 (s, 2.1H, CH₃iPr_{MAJ}), 1.30-1.29 (m, 6H, CH₂-hexyl), 0.95-0.82 (m, 3H, CH₃-hexyl).

**¹³C NMR** (100 MHz, CDCl₃), δ 136.65 (C^{IV}ₐᵣₒₘ), 128.69-128.28 (CHₐᵣₒₘ), 110.24 (C^{IV}iPr_{MAJ}), 110.15 (C^{IV}iPrₘᵢₙ), 96.60 (Cl_{MAJ}), 96.40 (Clₘᵢₙ), 92.71 (C6ₘᵢₙ), 92.68 (C6_{MAJ}), 74.35 (C2_{MAJ}), 74.32 (C2ₘᵢₙ), 72.52 (C3ₘᵢₙ), 72.03 (C3_{MAJ}), 69.74 (CH₂Ph_{MAJ}), 69.66 (CH₂Phₘᵢₙ), 69.49 (C4_{MAJ}), 69.05 (C4ₘᵢₙ), 61.26 (C5ₘᵢₙ), 60.79 (C5_{MAJ}), 31.12, 28.02, 25.99, 22.41 (CH₂-hexyl_{MAJ}), 31.17, 27.23, 25.93, 22.45 (CH₂-hexylₘᵢₙ), 26.17, 25.36 (CH₃iPr_{MAJ}), 26.13, 25.36 (CH₃iPrₘᵢₙ), 14.00 (CH₃-hexylₘᵢₙ), 13.98 (CH₃-hexyl_{MAJ}).

**HRMS (ESI)** [M+NH₄]⁺ cald for C₂₁H₃₅NO₇ *m*/*z* 427.243878; found *m*/*z* 427.243845; [M+Na]⁺ cald for C₂₁H₃₁NNaO₇ *m*/*z* 432.199273; found *m*/*z* 432.199176.

### Benzyl 2,3-O-isopropylidene[(1'R,1'S)(1-aminohex-1yl)]-β-L-ribopyranoside 6a and 6b

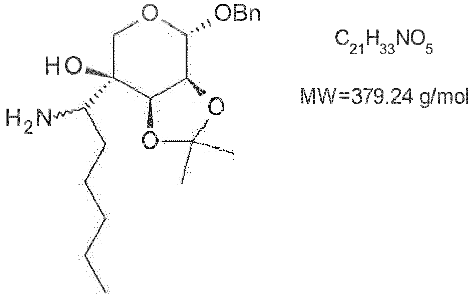

To a solution of the mixture of isomers **5a** and **5b** (289 mg, 0.71 mmol) in isopropanol (7.1 mL) were added glacial acetic acid (0.71 mL) and Raney Ni (106 mg). The mixture was left stirring under H₂ atmosphere for 5 days at RT. Then the mixture was filtered through a membrane, the retained catalyst was washed with MeOH and the solvents were evaporated to give the crude product as a thick, green oil. Flash column chromatography on silica gel (CH₂Cl₂/MeOH 9:1) gave an inseparable mixture of epimers **6a** and **6b** (228 mg, 85%) as a slightly green oil.

**¹H NMR** (400 MHz, isomers labelled as MAJ and min, CD₃OD), 8 7.43-7.28 (m, 5H, Hₐᵣₒₘ), 4.90 (d, 1H, H1, *J_{1,2}* = 3.3 Hz); 4.79 (d, 1H, CH₂Ph, *J* = 11.90 Hz), 4.61 (d, 1H, CH₂Ph, *J* = 11.90 Hz), 4.36 (d, 1H, H3, *J_{3,2}* = 5.8 Hz), 4.13 (dd, 1H, H2, *J_{2,1}* = 3.3 Hz, *J_{2,3} =* 5.8 Hz), 3.78 (d, 1H, H5_{b}, *J_{5b,5a}* = 12.0 Hz), 3.57 (d, 1H, H5ₐ, *J_{5a,5b}* = 12.0 Hz), 2.96 (dd, 0.7H, H6_{MAJ}, *J* = 2.4 Hz, *J =* 9.6 Hz), 2.81-2.79 (m, 0.3H, H6ₘᵢₙ), 1.69-1.26 (m, 14H, CH₂-hexyl, CH₃iPr), 0.95 (t, 3H, CH₃-hexyl, *J* = 6.6 Hz).

**¹³C NMR** (100 MHz, CD₃OD), δ 138.63 (C^{IV}ₐᵣₒₘ), 129.41, 129.19, 128.90 (CHₐᵣₒₘ), 110.96 (C^{IV}iPr_{MAJ}), 110.63 (C^{IV}iPrₘᵢₙ), 99.73 (Clₘᵢₙ), 99.56 (Cl_{MAJ}), 76.33 (C2), 76.09 (C3), 71.20 (C4), 70.76 (CH₂Ph), 64.84 (C5ₘᵢₙ), 63.52 (C5_{MAJ}), 58.39 (C6ₘᵢₙ), 57.59 (C6_{MAJ}), 32.95, 30.66, 27.24, 23.60 (CH₂-hexyl_{MAJ}), 31.66, 27.48 (CH₂-hexylₘᵢₙ), 26.92 (CH₃iPrₘᵢₙ), 26.84, 25.96 (CH₃iPr_{MAJ}), 14.39 (CH₃-hexyl).

**HRMS (ESI)** [M+H]⁺ cald for C₂₁H₃₄NO₅ *m*/*z* 380.243150; found *m*/*z* 380.243272.

### (3R, 4R, 5S, 6R/6S)-5-Hydroxymethyl-3,4-O-isopropylidene-6-C-pentyl-piperidine-3,4,5-triol 7a and 7b

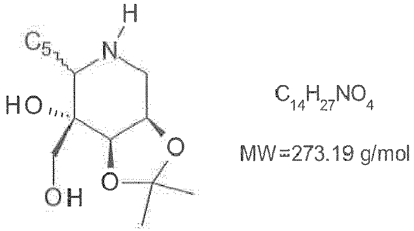

To a solution of the mixture of compounds **6a** and **6b** (367 mg, 0.968 mmol) in isopropanol (10 mL) were added glacial acetic acid (1 mL) and the catalyst 20% Pd(OH)₂ on C (150 mg). The mixture was left stirring under H₂ atmosphere for 2 d at RT. The mixture was then filtered through a membrane, the retained catalyst was washed with isopropanol and the filtrate was concentrated to give the crude product as a clean mixture of epimers at C-6 (7:3), **7a** and **7b** (264 mg, quant.), as a colorless oil.

**¹H NMR** (600 MHz, isomers labelled as MAJ and min, CD₃OD), 8 4.55 (dt, 0.7H, H2_{MAJ}, *J_{2,1b}* = *J_{2,1a}* = 3.9 Hz, *J_{2,3}* = 7.2 Hz), 4.49-4.46 (m, 0.3H, H2ₘᵢₙ), 4.29 (d, 0.7H, H3_{MAJ}, *J* = 7.2 Hz), 4.21 (dd, 0.3H, H3min, *J_{3,5}* = 1.2 Hz, *J_{3,2}* = 6.6 Hz), 3.72 (d, 0.3H, H6_{bmin}, *J* = 11.4 Hz), 3.66 (d, 0.3H, H6ₐₘᵢₙ, *J* = 11.4 Hz), 3.64 (d, 0.7H, H6_{bMAJ}, *J_{6b,6a}* = 11.4 Hz), 3.61 (d, 0.7H, H6_{aMAJ}, *J_{6a,6b}* = 11.4 Hz), 3.51 (dd, 0.7H, H1_{bMAJ}, *J_{1,2}* = 3.9 Hz, *J_{1b,1a}* = 13.5 Hz), 3.34 (dd, 0.3H, H1_{bmin}, *J_{1,2}* = 5.4 Hz, *J_{1b,1a}* = 13.2 Hz), 3.23 (dd, 0.7H, H5_{MAJ}, *J* = 3.6 Hz, *J =* 13.2 Hz), 3.21-3.18 (m, 0.3H, H5ₘᵢₙ), 3.18 (dd, 0.7H, H1_{aMAJ}, *J_{1a,2}* = 3.9 Hz, *J_{1a,1b}* = 13.5 Hz), 2.92 (dd, 0.3H, H1ₐₘᵢₙ, *J_{1a,2}* = 6.0 Hz, *J_{1a,1b} =* 13.2 Hz), 1.93-1.83 (m, 1H, CH₂-pentyl), 1.76-1.60 (m, 2H, CH₂-pentyl), 1.51 (s, 3H, CH₃iPr), 1.40-1.27 (m, 5H, CH₂-pentyl), 1.38 (s, 3H, CH₃iPr), 0.94 (t, 3H, CH₃-pentyl, *J* = 6.6 Hz).

**¹³C NMR** (62.5 MHz, CD₃OD):
MAJOR epimer: δ 110.68 (C^{IV}iPr), 75.20 (C3), 72.74 (C4), 70.79 (C2), 64.70 (C6), 54.83 (C5), 42.43 (C1), 32.84, 28.41, 26.53, 23.45 (CH₂-pentyl), 27.08, 24.58 (CH₃iPr), 14.35 (CH₃-pentyl).
MINOR epimer: δ 111.20 (C^{IV}iPr), 75.52 (C3), 72.82 (C4), 70.82 (C2), 65.27 (C6), 60.01 (C5), 41.61 (C1), 32.67,28.60,27.40 (CH₂-pentyl), 27.18,24.86 (CH₃iPr), 14.35 (CH₃-pentyl).

**HRMS (ESI)** [M+H]⁺ cald for C₁₄H₂₈NO₄ *m*/*z* 274.201285; found *m*/*z* 274.201459.

### Synthesis of compound 8 and epimer 13

Piperidine **7** (500 mg, 1.83 mmol) as a mixture of epimers **7a** and **7b** was dissolved in EtOH (15 mL). The solution was cooled down to 0°C, *N,N*-diisopropylethylamine (956 µL, 5.49 mmol, 3 eq) and benzyl chloroformate (392 µL, 2.75 mmol, 1.5 eq) were added. The mixture was stirred for 30 min at 0°C and 16 h at RT. The solvent was removed under vacuum and the residue was purified by flash column chromatography on silica gel (toluene/acetone 7/1) to give the separated epimers, major compound **13** (228 mg, 31%) and epimer **8** (132 mg, 18%) as mixtures of rotamers (65:35).

### (3R,4R,5S,6R)-1-Benzyloxycarbonyl-5-hydroxymethyl-3,4-O-isopropylidene-6-C-pentyl-piperidine-3,4,5-triol 8

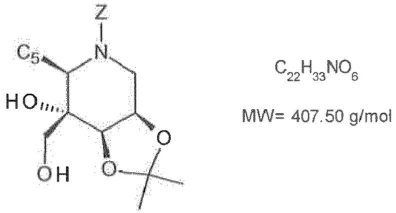

**¹H NMR** (400 MHz, rotamers labelled as MAJ and min, CDCl₃), δ 7.44-7.22 (m, 5H, Hₐᵣₒₘ), 5.25-5.03 (m, 2H, CH₂Ph), 4.40-4.23 (m, 2H, H1_{bmin}, H2, H5_{MAJ}), 4.22-4.16 (m, 0.35H, H5ₘᵢₙ), 4.12 (dd, 0.65H, H1_{bMAJ}, *J_{1b,2}* = 7.1 Hz, *J_{1b,1a} =* 13.8 Hz), 4.04-3.97 (m, 1H, H3), 3.83-3.64 (m, 2H, H6), 3.22 (s, 0.65H, OH_{MAJ}), 2.81-2.58 (m, 1.65H, H1ₐ, OH_{MAJ}), 2.58 (s, 0.35H, OHₘᵢₙ), 2.17 (br s, 0.35H, OHₘᵢₙ), 2.10-1.93 (m, 1H, H7_{b}), 1.47 (s, 3H, CH₃iPr), 1.31 (s, 3H, CH₃iPr), 1.41-1.02 (m, 7H, 3CH₂-pentyl, H7ₐ), 0.95-0.78 (m, 3H, CH₃-pentyl).

**¹³C NMR** (100 MHz, CDCl₃), δ 157.29 (CO_{MAJ}), 156.64 (COₘᵢₙ), 136.67 (C^{IV}ₐᵣₒₘₘᵢₙ), 136.56 (C^{IV}_{aromMAJ}), 129.10-127.70 (CHₐᵣₒₘ), 110.04 (C^{IV}iPrₘᵢₙ), 109.95 (C^{IV}iPr_{MAJ}), 76.34 (C3), 74.31 (C4_{MAJ}), 73.57 (C4ₘᵢₙ), 70.03 (C2_{MAJ}), 69.85 (C2ₘᵢₙ), 67.70 (CH₂Ph_{MAJ}), 67.62 (CH₂Phₘᵢₙ), 65.49 (C6_{MAJ}), 65.06 (C6ₘᵢₙ), 57.45 (C5ₘᵢₙ), 57.10 (C5_{MAJ}), 40.27 (C1_{MAJ}), 39.87 (C1ₘᵢₙ), 31.59 (CH₂-pentylₘᵢₙ), 31.54 (CH₂-pentyl_{MAJ}), 28.53, 26.20 (CH₃iPr_{MAJ}), 28.48, 26.27 (CH₃iPrₘᵢₙ), 25.99-25.68 (CH₂-pentyl, C7), 22.63 (CH₂-pentyl), 14.14 (CH₃-pentyl).

[α]_{D} = + 6.4 (20°C, c = 1.01, CHCl₃).

**HRMS (ESI)** [M+H]⁺ cald for C₂₂H₃₄NO₆ *m*/*z* 408.238064; found *m*/*z* 408.237733; [M+Na]⁺ cald for C₂₂H₃₃NNaO₆ *m*/*z* 430.220008; found *m*/*z* 430.220916.

### (3R,4R,5S,6S)-1-Benzyloxycarbonyl-5-hydroxymethyl-3,4-O-isopropylidene-6-C-pentyl-piperidine-3,4,5-triol 13

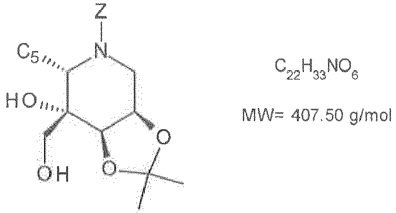

**¹H NMR** (400 MHz, rotamers labelled as MAJ and min, CDCl₃), δ 7.42-7.22 (m, 5H, Hₐᵣₒₘ), 5.21-5.04 (m, 2H, CH₂Ph), 4.40-4.25 (m, 2H, H2, H3), 4.25-4.09 (m, 1H, H1_{b}), 4.09-4.00 (m, 0.65H, H5_{MAJ}), 3.97-3.87 (m, 0.35H, H5ₘᵢₙ), 3.78 (br d, 1H, H6_{b}, *J_{6b},₆ₐ* = 11.2 Hz), 3.57-3.44 (m, 1H, H6ₐ), 3.37-3.18 (m, 1H, H1ₐ), 2.85-2.68 (m, 1H, OH), 2.58 (br s, 0.65H, OH_{MAJ}), 2.41 (br s, 0.35H, OHₘᵢₙ),1.69-1.11 (m, 14H, 3CH₂-pentyl, H7, 2CH₃iPr), 0.97-0.76 (m, 3H, CH₃-pentyl).

**¹³C NMR** (100 MHz, CDCl₃), δ 156.92 (CO_{MAJ}), 156.58 (COₘᵢₙ), 138.00 (C^{IV}_{aromMAJ}), 136.92 (C^{IV}ₐᵣₒₘₘᵢₙ), 129.17-127.90 (CHₐᵣₒₘ), 109.11 (C^{IV}iPrₘᵢₙ), 109.00 (C^{IV}iPr_{MAJ}), 75.47 (C2ₘᵢₙ), 75.27 (C2_{MAJ}), 73.46 (C3ₘᵢₙ), 73.15 (C3_{MAJ}), 72.84 (C4_{MAJ}), 72.42 (C4ₘᵢₙ), 67.58 (CH₂Phₘᵢₙ), 67.24 (CH₂Ph_{MAJ}), 65.59 (C6ₘᵢₙ), 65.22 (C6_{MAJ}), 54.38 (C5ₘᵢₙ), 54.06 (C5_{MAJ}), 41.90 (C1ₘᵢₙ) 41.80 (C1_{MAJ}), 32.13, 26.08 (CH₂-pentyl_{MAJ}), 28.76, 26.24 (CH₂-pentylₘᵢₙ), 28.09 (C7), 26.01 (CH₃iPr_{MAJ}), 25.88 (CH₃iPrₘᵢₙ), 24.53 (CH₃iPr), 22.72 (CH₂-pentyl), 14.19 (CH₃-pentyl).

**[α]_{D}** = + 52.2 (20°C, c = 1.38, MeOH).

**HRMS (ESI)** [M+H]⁺ cald for C₂₂H₃₄NO₆ *m*/*z* 408.238064; found *m*/*z* 408.237695; [M+Na]⁺ cald for C₂₂H₃₃NNaO₆ *m*/*z* 430.220008; found *m*/*z* 430.220420.

### (3R,4R,5S,6R)-1-Benzyloxycarbonyl-5-hydroxymethyl-3,4-O-isopropylidene-6-C-pentyl-piperidine-3,4,5-triol, 5,5'-thiocarbonyl derivative 9

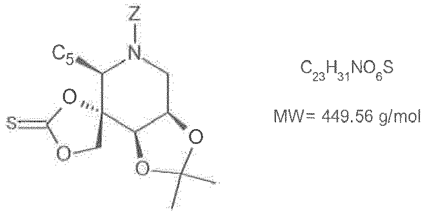

Diol **8** (78 mg, 0.191 mmol) was dissolved in anhydrous THF (2 mL) under argon. Thiocarbonyldiimidazole was added (51 mg, 0.288 mmol, 1.5 eq). The mixture was stirred under reflux until disappearance of the starting material on TLC (10 h), then cooled down to RT. The solvent was removed under vacuum and the residue was purified by flash column chromatography on silica gel (PE/EtOAc 7.5/1) to give 9 (74 mg, 86%) as a mixture of rotamers (6:4).

**¹H NMR** (400 MHz, rotamers labelled as MAJ and min, CDCl₃), δ 7.46-7.28 (m, 5H, Hₐᵣₒₘ), 5.25-5.07 (m, 2H, CH₂Ph), 4.85-4.72 (m, 1H, H6_{b}), 4.66-4.54 (m, 0.4H, H5ₘᵢₙ), 4.50-4.32 (m, 3.2H, H6ₐ, H3, H5_{MAJ}, H1_{bMAJ}), 4.24-4.14 (m, 1.4H, H2, H1_{bmin}), 2.94-2.82 (m, 0.4H, H1ₐₘᵢₙ), 2.75 (t, 0.6H, H1_{aMAJ}, *J₁ₐ,_{1b} = J_{1a,2}* = 11.2 Hz), 2.07-1.91 (m, 1H, H7_{b}), 1.51 (s, 3H, CH₃iPr), 1.36 (s, 3H, CH₃iPr), 1.40-1.08 (m, 7H, 3CH₂-pentyl, H7ₐ), 0.95-0.77 (m, 3H, CH₃-pentyl).

**¹³C NMR** (100 MHz, CDCl₃), δ 189.92 (CSₘᵢₙ), 189.78 (CS_{MAJ}), 155.52 (CO_{MAJ}), 155.47 (COₘᵢₙ), 136.31 (C^{IV}ₐᵣₒₘₘᵢₙ), 136.20 (C^{IV}_{aromMAJ}), 128.70-127.92 (CHₐᵣₒₘ), 111.12 (C^{IV}iPr), 87.71 (C4ₘᵢₙ), 87.52 (C4_{MAJ}), 74.60 (C2_{MAJ}), 74.35 (C2ₘᵢₙ), 72.95 (C6), 69.83 (C3ₘᵢₙ), 69.55 (C3_{MAJ}), 68.08 (CH₂Ph_{MAJ}), 67.89 (CH₂Phₘᵢₙ), 55.62 (C5_{MAJ}), 55.17 (C5ₘᵢₙ), 39.73 (C1ₘᵢₙ), 39.07 (C1_{MAJ}), 31.45, 26.32 (CH₂-pentylₘᵢₙ), 31.32, 25.45 (CH₂-pentyl_{MAJ}), 28.01 (CH₃iPr_{MAJ}), 27.81 (CH₃iPrₘᵢₙ), 26.14 (CH₃iPr), 25.96 (C7), 22.48 (CH₂-pentyl), 14.03 (CH₃-pentyl).

**HRMS (ESI)** [M+H]⁺ cald for C₂₃H₃₂NO₆S *m*/*z* 450.194485; found *m*/*z* 450.194331; [M+NH₄]⁺ cald for C₂₃H₃₅N₂O₆S *m*/*z* 467.221034; found *m*/*z* 467.221007; [M+Na]⁺ cald for C₂₃H₃₁NNaO₆S *m*/*z* 472.176429; found *m*/*z* 472.176275.

### (3R,4R,5R,6R)-1-Benzyloxycarbonyl-5-hydroxymethyl-3,4-O-isopropylidene-6-C-pentyl-piperidine-3,4-diol 10

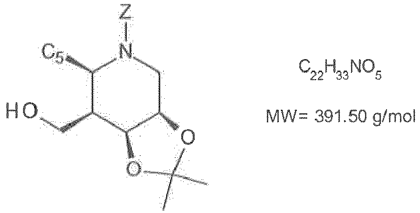

A solution of cyclic thiocarbonate 9 (72 mg, 0.160 mmol) and Bu₃SnH (86 µL, 0.320 mmol, 2 eq) was prepared in anhydrous and degassed toluene (3 mL). The solution was degassed 3 times by alternating vacuum and Ar bubbling. A solution of AIBN (3.2 mg, 0.019 mmol, 0.12 eq) in anhydrous and degassed toluene (4 mL) was then prepared. The solution was also degassed 3 times. Half of the AIBN solution was added to the solution of **9,** the mixture was stirred for 2 hours at RT, and then the second half of the solution was added. The mixture was stirred for 16 h under reflux. After cooling down to RT, the reaction mixture was washed twice with 10% aqueous KF (10 mL) and saturated aqueous NaHCO₃ (10 mL). The aqueous phases were collected and extracted 3 times with EtOAc (30 mL). The organic phases were combined and dried over MgSO₄ The solvent was removed under vacuum and the residue was purified by flash column chromatography on silica gel (PE/EtOAc 7/3) to give compound **10** (38 mg, 60%) as a colorless syrup containing traces of impurities from Bu₃SnH and as a mixture of rotamers (1:1).

**¹H NMR** (400 MHz, CDCl₃), δ 7.43-7.20 (m, 5H, Hₐᵣₒₘ), 5.20 (d, 0.5H, CH₂Ph, *J* = 12.4 Hz), 5.12 (s, 1H, CH₂Ph), 5.05 (d, 0.5H, CH₂Ph, *J* = 12.4 Hz), 4.42-4.26 (m, 2H, H3, H5), 4.22 (dd, 0.5H, 0.5H1_{b,} *J*_{1b,2} = 7.2 Hz, *J*_{1b,1a} = 13.6 Hz), 4.17-4.00 (m, 1.5H, H2, 0.5H1_{b}), 3.87-3.73 (m, 2H, 2H6), 2.82-2.64 (m, 1H, H1ₐ), 2.26-2.13 (m, 1H, H4), 2.03-1.81 (m, 2H, H7_{b}, OH), 1.49 (s, 3H, CH₃iPr), 1.33 (s, 3H, CH₃iPr), 1.43-1.05 (m, 7H, 3CH₂-pentyl, H7ₐ), 0.90-0.78 (m, 3H, CH₃-pentyl).

**¹³C NMR** (100 MHz, CDCl₃), δ 155.76, 155.48 (CO), 136.78, 136.74 (C^{IV}ₐᵣₒₘ), 128.61-127.76 (CHₐᵣₒₘ), 109.54 (C^{IV}iPr), 73.41, 73.31 (C3), 71.22, 70.98 (C2), 67.45, 67.31 (CH₂Ph), 61.82, 61.75 (C6), 51.17, 50.99 (C5), 42.24, 41.86 (C4), 39.84, 39.49 (C1), 31.70, 25.90, 25.81, 22.66 (CH₂-pentyl), 28.77, 26.29, 26.24 (CH₃iPr), 26.93, 26.78 (C7), 14.15, 14.14 (CH₃-pentyl).

**HRMS (ESI)** [M+H]⁺ cald for C₂₂H₃₄NO₅ *m*/*z* 392.243150; found *m*/*z* 392.243160; [M+Na]⁺ cald for C₂₂H₃₃NNaO₅ *m*/*z* 414.225094; found *m*/*z* 414.224975.

### (3R,4R,5R,6R)-5-Hydroxymethyl-3,4-O-isopropylidene-6-C-pentyl-piperidine-3,4-diol 11

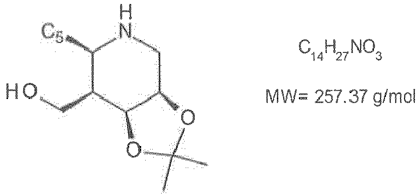

The *N-*protected compound 10 (38 mg, 0.097 mmol) was dissolved in isopropanol (6 mL). 10% Pd on C (80 mg) was added. The reaction was stirred at RT under H2 atmosphere until completion and the mixture was filtered through a membrane. The retained catalyst was washed with MeOH and the filtrate was concentrated under high vacuum to give the deprotected compound **11** (24 mg, 96%).

**¹H NMR** (250 MHz, CD₃OD), δ 4.38 (dd, 1 H, H3, *J₃,₄ =* 3.3 Hz, *J_{3,2} =* 5.8 Hz), 4.14 (dt, 1 H, H2, *J*_{2,3} = *J*_{2,1b} = 5.8, *J*_{2,1a} = 7.0 Hz), 3.68 (d, 2H, 2H6, *J*_{6,4} *=* 7.5 Hz), 2.99 (ddd, 1H, H5, *J₅,₇ =* 2.8 Hz, *J_{5,4} =* 6.3 Hz, *J₅,₇ =* 9.2 Hz), 2.79 (dd, 1H, H1_{b}, *J_{1b,2} =* 5.8 Hz, *J_{1b,1a}* = 13.0 Hz), 2.68 (dd, 1H, H1ₐ, *J_{1a,2}* = 7.0 Hz, *J_{1a,1b}* = 13.0 Hz), 2.16 (ddt, 1H, H4, *J_{4,3} =* 3.3 Hz, *J_{4,5} =* 6.3 Hz, *J_{4,6} =* 7.5 Hz), 1.92-1.74 (m, 1H, H7_{b}), 1.46 (s, 3H, CH₃iPr), 1.31 (s, 3H, CH₃iPr), 1.56-1.14 (m, 7H, 3CH₂-pentyl, H7ₐ), 0.97-0.87 (m, 3H, CH₃-pentyl).

**¹³C NMR** (100 MHz, CD₃OD), δ 109.81 (C^{IV}iPr), 73.90 (C3), 73.32 (C2), 61.76 (C6), 52.70 (C5), 42.84 (C1), 42.46 (C4), 33.09, 27.46, 23.62 (CH₂-pentyl), 29.26 (C7), 28.06, 25.45 (CH₃iPr), 14.40 (CH₃-pentyl).

**[α]_{D}** = + 31.7 (20°C, c = 1, MeOH).

**HRMS (ESI)** [M+H]⁺ cald for C₁₄H₂₈NO₃ *m*/*z* 258.206370; found *m*/*z* 258.206607.

### (3R,4R,5R,6R)-5-Hydroxymethyl-6-C-pentyl-piperidine-3,4-diol or (6R)-6-C-pentyl 4-epi-isofagomine 12

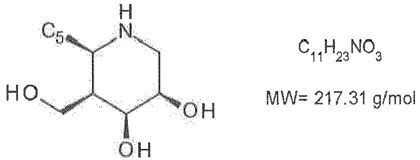

The protected compound **11** (23 mg, 0.089 mmol) was dissolved in a mixture of dioxane/H₂O (5/3; 8 mL) and Dowex ion-exchange resin (H⁺ form, 50WX8, 1 mL) was added. The reaction was stirred for 18 h at RT. The mixture of resin and solvents was poured into a column and washed with dioxane/H₂O (1/1, 50 mL), then with H₂O (50 mL). Elution with aqueous 0.5 N NH₄OH (80 mL) and evaporation of solvent under high vacuum gave the expected pure compound **12** (16 mg, 84%).

**¹H NMR** (250 MHz, CD₃OD), δ 3.90 (dd, 1H, H6_{b}, *J_{6b,4} =* 3.5 Hz, *J_{6b},₆ₐ =* 11.5 Hz), 3.87-3.84 (m, 1H, H3), 3.80 (dd, 1H, H6ₐ, *J_{6a,4} =* 4.5 Hz, *J_{6a,6b} =* 11.5 Hz), 3.75-3.68 (m, 1H, H2), 3.00 (dd, 1H, H1_{b}, *J_{1b,2}* = 2.8 Hz, *J_{1b,1a}*= 13.3 Hz), 2.73 (d, 1H, H1ₐ, *J_{1a,1b}=* 13.3 Hz), 2.68-2.64 (m, 1H, H5), 1.86 (quint, 1H, H4, *J₄,₃* = *J_{4,6}* = *J_{4,5}* = 4.3 Hz), 1.72-1.53 (m, 2H, 2H7), 1.49-1.26 (m, 6H, CH₂-pentyl), 0.97-0.87 (m, 3H, CH₃).

**¹³C NMR** (100 MHz, CD₃OD), δ 72.04 (C3), 68.92 (C2), 58.63 (C5), 58.15 (C6), 50.76 (C1), 44.08 (C4), 33.11, 27.39, 23.63 (CH₂-pentyl), 32.96 (C7), 14.39 (CH₃).

**[α]_{D} = -** 9.5 (20°C, c = 1, MeOH).

**HRMS (ESI)** [M+H]⁺ cald for C₁₁H₂₄NO₃ *m*/*z* 218.175070; found *m*/*z* 218.175272.

### Example 3: Inhibition of wild type beta₋galactosidase activity in human peripheral blood mononuclear cell lysate by 4-epi-isofagomine derivatives of general_formula (I), and epi-isofagomine.

4-epi-isofagomine derivatives of general formula (I) were synthesized according to the protocol disclosed on example 2. Two isomeric forms were synthesized, one with a pentyl group in the R configuration, (2R,3R,4S,5R)-3-hydroxymethyl-2-pentylpiperidine-4,5-diol, and another one with a pentyl group in the S configuration, (2R,3R,4S,5S)-3-hydroxymethyl-2-pentylpiperidine-4,5-diol. Both 4-epi-isofagomine derivatives of general formula (I) were tested for their beta-galactosidase inhibitory activity.

### Experimental Protocol

Samples of human peripheral blood mononuclear cell lysate (2 x 10⁶ cells per assay) were incubated with graded concentrations of compound in 25 mM sodium phosphate, pH 7.3, supplemented with 0.5% Triton X-100 and protease inhibitors, and 0.8 mM of 4-methylumbelliferyl β-D-galactopyranoside. After an incubation of 2 hours at 37°C, the enzymatic reactions were stopped by the addition of 0.15 M glycine, pH 10.2, and fluorescence was measured at 445 nm using an excitation wavelength of 365 nm. The relative fluorescence corresponding to (2R,3R,4S,5R)-3-hydroxymethyl-2-pentylpiperidine-4,5-diol, (squares), (2R,3R,4S,5S)-3-hydroxymethyl-2-pentylpiperidine-4,5-diol (circles) and epi-isofagomine (triangles) is shown on figure 2.

### Results

The 4-epi-isofagomine derivatives of general formula (I), (2R,3R,4S,5R)-3-hydroxymethyl-2-pentylpiperidine-4,5-diol is a strong inhibitor of human beta-galactosidase activity while the S isomer, (2R,3R,4S,5S)-3-hydroxymethyl-2-pentylpiperidine-4,5-diol is 150-fold less potent. This latter is however still a potent inhibitor of human beta-galactosidase activity, as well as the compound epi-isofagomine (Fig. 2).

IC50 of (2R,3R,4S,5R)-3-hydroxymethyl-2-pentylpiperidine-4,5-diol, (2R,3R,4S,5S)-3-hydroxymethyl-2-pentylpiperidine-4,5-diol, and epi-isofagomine are 0.01 µM, 1.5 µM and 0.8 µM, respectively.

### Example 4: (2R,3R,4S,5R)-3-hydroxymethyl-2-pentylpiperidine-4,5-diol is a potent and specific inhihitor of wild type human beta-galactosidase activity.

### Experimental Protocol

Human peripheral blood mononuclear cell lysate was used as a source of human beta-galactosidase, and 4-methylumbelliferyl β-D-galactopyranoside was used as a substrate The activities of the various enzymes were revealed with the corresponding colorimetric or fluorogenic substrates. IC50 values indicated in table 1 are the concentrations of (2R,3R,4S,5R)-3-hydroxymethyl-2-pentylpiperidine-4,5-diol compound required to inhibit by half the different enzymatic activities.

**Table 1:**

| Human enzymes | IC50 (2R,3R,4S,5R)-3-hydroxymethyl-2-pentylpiperidine-4,5-diol (µM) |
|---|---|
| Beta-galactosidase | 0.02 |
| Alpha-galactosidase | 150 |
| Alpha-glucosidase | >160 |
| Beta-glucosidase | 60 |
| Alpha-hexosaminidase | >160 |
| Beta-hexosaminidase | >160 |
| Beta-glucuronidase | >160 |

### Results

The 4-epi-isofagomine derivatives of general formula (I), (2R,3R,4S,5R)-3-hydroxymethyl-2-pentylpiperidine-4,5-diol is a strong beta-galactosidase inhibitor (IC50 = 20 nM; Table 1). The specificity of said compound for other closely related glycosidases was evaluated and revealed that it weakly inhibited the activity of human beta-glucosidase and alpha-galactosidase (IC50 = 60 and 150 µM, respectively; Table 1) and any of the other four human glycosidases tested (IC50 > 160 µM; Table 1). These results indicated that (2R,3R,4S,5R)-3-hydroxymethyl-2-pentylpiperidine-4,5-diol is highly specific for human beta-galactosidase. The exquisite specificity of said compound for beta-galactosidase would thus limit the *in vivo* side effects due to the blockade of other glycosidases.

### Example 5: (2R,3R,4S,5R)-3-hydroxymethyl-2-pentylpiperdine-4,5-diol prevents heat denaturation of human beta-galactosidase.

The capacity of a compound of protecting an enzyme from denaturation predicts to some extent its pharmacological chaperone activity, *i*.*e*. its capacity of restoring enzymatic activity in patient cells exposed to the compound. The Applicant has evaluated whether (2R,3R,4S,5R)-3-hydroxymethyl-2-pentylpiperidine-4,5-diol can prevent heat denaturation of human beta-galactosidase following a procedure described by Suzuki et al 2014.

### Experimental Protocol

Samples of human peripheral blood mononuclear cell lysate (2 x 10⁶ cells) in phosphate buffer, pH 7.3, 0.5% Triton X-100 and protease inhibitors were incubated at 48°C for 0, 5, 10, 20, and 40 minutes in the presence of graded concentrations of (2R,3R,4S,5R)-3-hydroxymethyl-2-pentylpiperidine-4,5-diol. The samples were then diluted in citrate buffer, pH 4.3, and 0.25 mM of 4-methylumbelliferyl β-D-galactopyranoside. After an incubation of 17 hours at 37°C, the reaction was stopped by the addition of glycine 0.15 M, pH 10.2. Fluorescence was determined at 445 nm using an excitation wavelength at 365 nm. The tested concentrations of (2R,3R,4S,5R)-3-hydroxymethyl-2-pentylpiperidine-4,5-diol, are 20 µM (square), 2 µM (triangle), 0.2 µM (diamond), 0.02 µM (circle) and 0 µM (line).

### Results

Beta-galactosidase activity in peripheral blood mononuclear cell lysate treated at 48°C in absence of compound was reduced by 50% in 3 minutes (Fig. 3). Addition of increasing concentrations of (2R,3R,4S,5R)-3-hydroxymethyl-2-pentylpiperidine-4,5-diol progressively reduced the loss of beta-galactosidase activity. In the presence of 0.02, 0.2, 2 and 20 µM of said compound, loss of 50% of the beta-galactosidase activity was attained in 10,25 and >40 minutes, respectively (Table 2 and Fig. 3).

**Table 2:**

| (2R,3R,4S,5R)-3-hydroxymethyl-2-pentylpiperidine-4,5-diol | beta-galactosidase activity % | | | | |
|---|---|---|---|---|---|
| Concentration (µM) | 0 minute | 5 minutes | 10 minutes | 20 minutes | 40 minutes |
| 0 | 100 | 29 | 13 | 9.1 | 5.5 |
| 0.02 | 100 | 70 | 46 | 23 | 11 |
| 0.2 | 100 | 81 | 72 | 56 | 33 |
| 2 | 100 | 82 | 84 | 73 | 70 |
| 20 | 100 | 93 | 95 | 85 | 87 |

Upon treatment of peripheral blood mononuclear cell lysate at 48°C for 10 minutes, 84% and 95% of beta-galactosidase activity was spared by the presence of 2µM and 20 µM of (2R,3R,4S,5R)-3-hydroxymethyl-2-pentylpiperidine-4,5-diol, respectively. These results indicate that this compound prevents heat denaturation of beta-galactosidase activity.

These results indicate that (2R,3R,4S,5R)-3-hydroxymethyl-2-pentylpiperidine-4,5-diol prevents heat denaturation of human beta-galactosidase and is therefore endowed with beta-galactosidase chaperone activity.

### Example 6: (2R,3R,4S,5R)-3-hydroxymethyl-2-pentylpiperidine-4,5-diol promotes increase of mutated beta-galactosidase activity in fibroblasts of GM1-gangliosidosis patients.

(2R,3R,4S,5R)-3-hydroxymethyl-2-pentylpiperidine-4,5-diol was tested for its capacity to increase mutated beta-galactosidase activity levels in fibroblasts from Morquio disease type B and GM1-gangliosidosis patients.

### Experimental Protocol

GM02439 and GM05335 fibroblasts (Coriell Institute) were cultured for 4 days in the presence or the absence of 2 µM of (2R,3R,4S,5R)-3-hydroxymethyl-2-pentylpiperidine-4,5-diol. Cells were lysed in 100 mM sodium citrate, pH 4.8, supplemented with 1% Triton X-100 and protease inhibitors. B eta-galactosidase and beta-hexosaminidase activities were then determined in the cell lysates using fluorescence and colorimetric assays, respectively. Results presented are means of 4 values obtained from independent cultures. Percent of deviation of each value is <30% from the mean. Hatched and empty bars: beta-galactosidase and beta-hexosaminidase activities, respectively.

### Results

This treatment results in an increase of mutated beta-galactosidase activity of 6.5- and 19-fold in the juvenile GM1-gangliosidosis cell line GM02439 and the infantile GM1-gangliosidosis cell line GM05335, respectively (Fig. 4). In contrast, there is no increase of the beta-hexosaminidase activity at all.

## Claims

1. A 4-epi-isofagomine derivative of general formula (I): 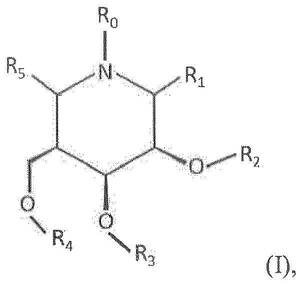 and pharmaceutically acceptable salts thereof wherein:
R₀ is selected from the group comprising hydrogen, C₁-C₃ alkyl, C₂-C₆ alkenyl, C₂-C₆ alkynyl, C₃-C₆ cycloalkyl, C₃-C₁₈ heterocycle, C₆ aryl, and C₇-C₈ arylalkyl;
R₁ is selected from the group comprising hydrogen, C₁-C₆ alkyl, C₂-C₆ alkenyl, C₂-C₆ alkynyl, C₃-C₆ cycloalkyl, C₃-C₁₈ heterocycle, C₆ aryl, and C₇-C₈ arylalkyl;
R₂, R₃ and R₄ are each independently selected from the group comprising hydrogen, C₁-C₄ alkyl, saturated or non-saturated C₁-C₄ acyl, C₂-C₄ alkenyl, and C₂-C₄ alkynyl;
R₅ is selected from the group comprising hydrogen, substituted or unsubstituted C₁-C₁₂ alkyl, C₂-C₁₂ alkenyl, C₂-C₁₂ alkynyl, C₃-C₁₂ cycloalkyl, C₃-C₁₈ heterocycle, C₆-C₁₈ aryl, C₇-C₁₈ arylalkyl,
with the proviso that when R₅ is hydrogen, at least one of R₀, R₁, R₂, R₃ and R₄ is not hydrogen.

2. The 4-epi-isofagomine derivative of general formula (I), or a pharmaceutically acceptable salt thereof, according to claim 1, wherein:
R₀ is hydrogen;
R₁ is hydrogen;
R₂, R₃ and R₄ are each independently selected from the group comprising C₁-C₄ alkyl, saturated or non-saturated C₁-C₄ acyl, C₂-C₄ alkenyl, and C₂-C₄ alkynyl; and
R₅ is selected from the group comprising hydrogen, substituted or unsubstituted C₁-C₁₂ alkyl, C₂-C₁₂ alkenyl, C₂-C₁₂ alkynyl, C₃-C₁₂ cycloalkyl, C₃-C₁₈ heterocycle, C₆-C₁₈ aryl, C₇-C₁₈ arylalkyl

3. The 4-epi-isofagomine derivative of general formula (I), or a pharmaceutically acceptable salt thereof, according to claim 1, wherein R₀, R₁, R₂, R₃ and R₄ are hydrogen and R₅ is selected from the group comprising substituted or unsubstituted C₁-C₁₂ alkyl, C₂-C₁₂ alkenyl, C₂-C₁₂ alkynyl, C₃-C₁₂ cycloalkyl, C₃-C₁₈ heterocycle, C₆-C₁₈ aryl, C₇-C₁₈ arylalkyl.

4. The 4-epi-isofagomine derivative of general formula (I), or a pharmaceutically acceptable salt thereof, according to claim 2 or 3, wherein R₅ is selected from the group comprising pentyl, hexyl, heptyl, octyl, nonyl, ethylphenyl, 2-(4-trifluoromethylphenyl)ethyl, 2-ethylhexyl, cyclopropylmethyl, cyclohexylmethyl, cycloheptylmethyl, 5-hexenyl, 5-hexinyl, 5-acetamido-1-hydroxypent-2-yl, and 6-acetamido-hexyl.

5. The 4-epi-isofagomine derivative of general formula (I), or a pharmaceutically acceptable salt thereof, according to claim 1, selected from the group comprising:
(2R,3R,4S,5R)-3-Hydroxymethyl-2-pentylpiperidine-4,5-diol;
(2R,3R,4S,5R)-3-Hydroxymethyl-2-hexylpiperidine-4,5-diol;
(2R,3R,4S,5R)-3-Hydroxymethyl-2-heptylpiperidine-4,5-diol;
(2R,3R,4S,5R)-3-Hydroxymethyl-2-octylpiperidine-4,5-diol;
(2R,3R,4S,5R)-3-Hydroxymethyl-2-nonylpiperidine-4,5-diol;
(2R,3R,4S,5R)-3-Hydroxymethyl-2-ethylphenylpiperidine-4,5-diol;
(2R,3R,4S,5R)-3-Hydroxymethyl-2-[2-(4-trifluoromethylphenyl)ethyl]piperidine-4,5-diol;
(2R,3R,4S,5R)-3-Hydroxymethyl-2-(2-ethylhexyl)piperidine-4,5-diol;
(2R,3R,4S,5R)-3-Hydroxymethyl-2-cyclopropylmethylpiperidine-4,5-diol;
(2R,3R,4S,5R)-3-Hydroxymethyl-2-cyclohexylmethylpiperidine-4,5-diol;
(2R,3R,4S,5R)-3-Hydroxymethyl-2-cycloheptylmethylpiperidine-4,5-diol;
(2R,3R,4S,5R)-3-Hydroxymethyl-2-(5-hexenyl)piperidine-4,5-diol;
(2R,3R,4S,5R)-3-Hydroxymethyl-2-(5-hexinyl)piperidine-4,5-diol;
(2R,3R,4S,5R)-3-Hydroxymethyl-2-(5-acetamido-1-hydroxypent-2-yl)piperidine-4,5-diol; and
(2R,3R,4S,5R)-3-Hydroxymethyl-2-(6-acetamido-hexyl)piperidine-4,5-diol.

6. The 4-epi-isofagomine derivative of general formula (I), or a pharmaceutically acceptable salt thereof, according to any of the preceding claims, wherein said 4-epi-isofagomine derivative is an inhibitor of wild type beta-galactosidase activity.

7. The 4-epi-isofagomine derivative of general formula (I), or a pharmaceutically acceptable salt thereof, according to claim 6, wherein said 4-epi-isofagomine derivative has an IC50 of less than about 10 µM.

8. A 4-epi-isofagomine derivative according to any of the preceding claims, for use as a medicament.

9. A 4-epi-isofagomine derivative according to any of the preceding claims, for use in the treatment and/or prevention of Morquio disease type B, GM1-gangliosodosis, Krabbe disease, galactosemia, Tay-Sachs disease, Sandhoff-Jatzkewitz's disease, Fabry disease, metachromatic leukodystrophy, and/or Morquio disease type A.

10. A 4-epi-isofagomine derivative according to any of the preceding claims, for use in the treatment and/or prevention of diseases caused by dysfunctions in beta-galactosidase.

11. A 4-epi-isofagomine derivative according to claims 1-9, for use in the treatment and/or prevention of lysosomal storage diseases, wherein said lysosomal storage diseases are selected from the group of pathologies caused by dysfunctions in beta-galactosidase, alpha-galactosidase, galactocerebrosidase, alpha-glucosidase, alpha-mannosidase, beta-mannosidase, alpha-fucosidase galactose-1-phosphate uridylyltransferase, galactosylceramide beta-galactosidase and galactosyltransferases.

12. A pharmaceutical composition comprising a 4-epi-isofagomine derivative according to any of the preceding claims and a pharmaceutically acceptable carrier, diluent or excipient.

13. The pharmaceutical composition according to claim 12, further comprising at least one or more additional agents selected from the group comprising non-competitive chaperone compounds and GM1-ganglioside synthesis inhibitors.

14. The 4-epi-isofagomine derivative of general formula (I), or a pharmaceutically acceptable salt thereof according to claims 1, wherein said 4-epi-isofagomine derivative prevents denaturation of beta-galactosidase.
